(19) Europäisches Patentamt
European Patent Office
Office européen des brevets

(11) Veröffentlichungsnummer: **0 086 748**
**B1**

(12) # EUROPÄISCHE PATENTSCHRIFT

(45) Veröffentlichungstag der Patentschrift: **13.11.85**

(51) Int. Cl.⁴: **C 07 D 275/06**, A 01 N 43/00

(21) Anmeldenummer: **83810052.7**

(22) Anmeldetag: **07.02.83**

(54) **3-Amidino-benzisothiazol-1,1-dioxide, Verfahren zu ihrer Herstellung und ihre Verwendung in der Schädlingsbekämpfung.**

(30) Priorität: **12.02.82 CH 888/82**
**07.01.83 CH 87/83**

(43) Veröffentlichungstag der Anmeldung:
**24.08.83 Patentblatt 83/34**

(45) Bekanntmachung des Hinweises auf die Patenterteilung:
**13.11.85 Patentblatt 85/46**

(84) Benannte Vertragsstaaten:
**AT BE CH DE FR IT LI NL**

(56) Entgegenhaltungen:
**EP - A - 0 033 984**
**DE - A - 1 670 797**
**US - A - 2 751 392**

(73) Patentinhaber: **CIBA-GEIGY AG, Postfach, CH-4002 Basel (CH)**

(72) Erfinder: **Drabek, Jozef, Dr., Benkenstrasse 12, CH-4104 Oberwil (CH)**

**EP 0 086 748 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Aus der US-A-2 751 392 sind bereits Aminderivate des Benzisothiazol-1,1-dioxids bekannt, denen insbesondere eine analgetische und Antihistaminwirkung zueigen ist. Es sind ferner aus der DE-A-1 670 797 Dialkylaminobenzisothiazol-1,1-dioxide bekanntgeworden, für welche eine Verwendung als Zwischenprodukte u. a. für Farbstoffe angegeben ist.

Die vorliegende Erfindung dagegen betrifft 3-Amidino-benzisothiazol-1,1-dioxide, Verfahren zu ihrer Herstellung und ihre Verwendung in der Schädlingsbekämpfung.

Die 3-Amidino-benzisothiazol-1,1-dioxide haben die Formel

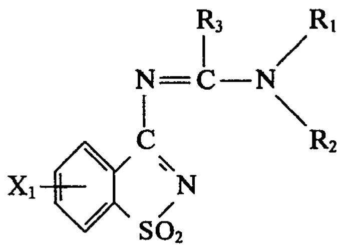

(I)

worin

$R_1$ und $R_2$ je $C_2$—$C_5$-Alkyl oder $C_2$—$C_5$-Alkenyl,
$R_3$ Wasserstoff oder Methyl oder
$R_1$ und $R_2$ zusammen eine gegebenenfalls durch O, S oder NH unterbrochene $C_3$—$C_6$-Alkylenkette oder
$R_1$ und $R_3$ zusammen $-CH_2-CH_2-CH_2-$ und
$X_1$ Wasserstoff, Halogen $C_1$—$C_5$-Alkyl, Halogen-$C_1$—$C_5$-alkyl oder $C_1$—$C_5$-Alkoxy bedeuten.

Unter Halogen ist dabei Fluor, Chlor, Brom oder Jod, insbesondere aber Fluor und Chlor, zu verstehen.

Die bei $R_1$, $R_2$ und $X_1$ stehenden Alkyl-, Halogenalkyl-, Alkenyl- oder Alkoxygruppen können geradkettig oder verzweigt sein. Beispiele solcher Gruppen sind z. a.: Methyl, Trifluormethyl, Methoxy, Äthyl, Äthoxy, Propyl, Propoxy, Isopropyl, Isopropoxy, n-, i-, sek.-, tert.-Butyl, n-Pentyl und dessen Isomere, Vinyl, 1-Propenyl, 2-Propenyl, Isopropenyl, 3-Butenyl oder

$$-CH_2-CH_2-CH=CH-CH_3$$

Die bevorzugte Alkenylgruppe bei $R_1$ und $R_2$ ist 2-Propenyl.

Beispiele von Alkylenketten bei $R_1$ und $R_2$ sind: Propylen, Butylen, Pentylen und Hexylen, welche ein- oder mehrfach, insbesondere aber einfach, durch O, S oder —NH unterbrochen sein können. Bevorzugte Alkylenketten bei $R_1$ und $R_2$ sind Pentylen,

$$-CH_2-CH_2-O-CH_2-CH_2-$$

$$-CH_2-CH_2-S-CH_2-CH_2-$$

oder

$$-CH_2-CH_2-NH-CH_2-CH_2-$$

Bevorzugt sind Verbindungen der Formel I, worin

$R_1$ und $R_2$ je $C_1$—$C_5$-Alkyl oder $C_2$—$C_5$-Alkenyl,
$R_3$ Wasserstoff oder Methyl oder
$R_1$ und $R_2$ zusammen eine gegebenenfalls durch O, S oder NH unterbrochene $C_3$—$C_6$-Alkylenkette oder
$R_1$ und $R_3$ zusammen $-CH_2-CH_2-CH_2-$ und
$X_1$ Wasserstoff, Halogen, $C_1$—$C_5$-Alkyl oder $C_1$—$C_5$-Alkoxy bedeuten.

Insbesondere bevorzugt sind Verbindungen der Formel I, worin $R_1$ und $R_2$ je $C_1$—$C_4$-Alkyl oder 2-Propenyl oder $R_1$ und $R_2$ zusammen

$$-CH_2-CH_2-CH_2-CH_2-CH_2-$$

$$-CH_2-CH_2-O-CH_2-CH_2-$$

$$-CH_2-CH_2-S-CH_2-CH_2-$$

oder

$—CH_2—CH_2—NH—CH_2—CH_2—$

$R_3$ Wasserstoff und $X_1$ Wasserstoff, Halogen, Methyl, Trifluormethyl oder Methoxy bedeuten oder Verbindungen der Formel I, worin $R_1$ und $R_2$ je $C_1$–$C_4$-Alkyl oder 2-Propenyl oder $R_1$ und $R_2$ zusammen

$—CH_2—CH_2—CH_2—CH_2—CH_2—$

$—CH_2—CH_2—O—CH_2—CH_2—$

$—CH_2—CH_2—S—CH_2—CH_2—$

oder

$—CH_2—CH_2—NH—CH_2—CH_2—$

$R_3$ Wasserstoff und $X_1$ Wasserstoff, Chlor, Methyl oder Methoxy bedeuten.

Ganz besonders bevorzugt sind Verbindungen der Formel I, worin $R_1$ und $R_2$ je Methyl, n-Butyl oder 2-Propenyl oder $R_1$ und $R_2$ zusammen

$—CH_2—CH_2—CH_2—CH_2—CH_2—$

oder

$—CH_2—CH_2—O—CH_2—CH_2—$

$R_3$ Wasserstoff und $X_1$ Wasserstoff oder Chlor bedeuten oder Verbindungen der Formel I, worin $R_1$ und $R_2$ je Methyl, n-Butyl oder 2-Propenyl oder $R_1$ und $R_2$ zusammen

$—CH_2—CH_2—CH_2—CH_2—CH_2—$

oder

$—CH_2—CH_2—O—CH_2—CH_2—$

$R_3$ und $X_1$ je Wasserstoff bedeuten.

Die Verbindungen der Formel I werden nach an sich bekannten Methoden z. B. wie folgt hergestellt:

1. 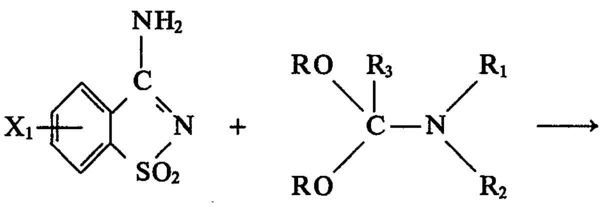 (I)

(II) (III)

2. 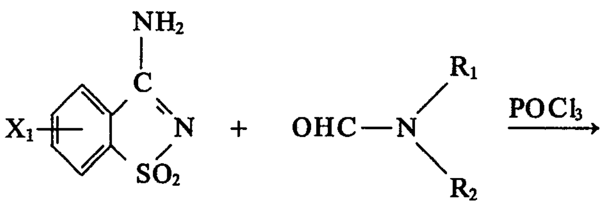

(I)

(II) (IV)

In den Formeln II bis IV haben $R_1$, $R_2$, $R_3$ und $X_1$ die für die Formel I angegebene Bedeutung, und R steht für $C_1$–$C_5$-Alkyl, insbesondere für Methyl oder Äthyl. In den bevorzugten Benzisothiazolen der Formel II bedeutet $X_1$ Wasserstoff, Chlor, Methyl oder Methoxy, insbesondere aber Wasserstoff oder Chlor. Beispiele solcher Benzisothiazole sind u. a.: 3-Aminobenzisothiazol-1,1-dioxid; 3-Amino-4-chlorbenzisothiazol-1,1-dioxid; 3-Amino-4-methylbenzisothiazol-1,1-dioxid und 3-Amino-4-methoxybenzisothiazol-1,1-dioxid. In den bevorzugten Acetalen der Formel III bedeuten $R_1$ und $R_2$ je

$C_1$–$C_4$-Alkyl oder 2-Propenyl oder $R_1$ und $R_2$ zusammen

$$-CH_2-CH_2-CH_2-CH_2-CH_2-$$
$$-CH_2-CH_2-O-CH_2-CH_2-$$
$$-CH_2-CH_2-S-CH_2-CH_2-$$

oder

$$-CH_2-CH_2-NH-CH_2-CH_2-$$

$R_3$ Wasserstoff und R Äthyl. Beispiele solcher Acetale sind u. a. Dimethylformamiddiäthylacetal, Di-n-butylformamiddiäthylacetal sowie Acetale der Formeln

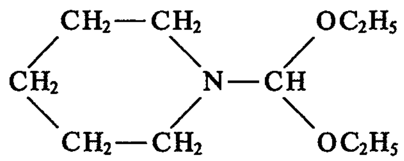

und

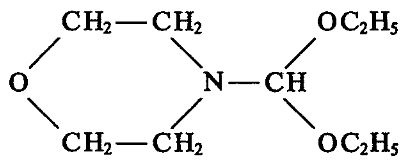

Die Verfahren werden bei normalem Druck, bei einer Temperatur von 0° bis 160°C, vorzugsweise bei 60° bis 140°C und gegebenenfalls in einem Lösungs- oder Verdünnungsmittel durchgeführt.

Als Lösungs- oder Verdünnungsmittel eignen sich z. B. Äther und ätherartige Verbindungen wie Diäthyläther, Di-isopropyläther, Dioxan, Tetrahydrofuran; aliphatische und aromatische Kohlenwasserstoffe, insbesondere Benzol, Toluol, Xylole; und Ketone wie Aceton, Methyläthylketon und Cyclohexanon.

Die Ausgangsstoffe der Formeln II bis IV sind bekannt bzw. können analog bekannten Methoden hergestellt werden.

So wird das Verfahren zur Herstellung von bevorzugten Benzisothiazolen der Formel II in der Europäischen Patentanmeldung Nr. 0 033 984 und in der japanischen Patentanmeldung Nr. 73 /24 735 und von Acetalen der Formel III in Liebigs Annalen der Chemie 641, 1, 1961, beschrieben.

Die Verbindungen der Formel I eignen sich zur Bekämpfung von Schädlingen an Tieren und Pflanzen.

Insbesondere eignen sich die Verbindungen der Formel I zur Bekämpfung von Insekten, z. B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und Milben und Zecken der Ordnung Acarina.

Vor allem eignen sich die Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z. B. Spodoptera littoralis und Heliothis virescens), Gemüsekulturen (z. B. Leptinotarsa decemlineata und Myzus persicae) und Reiskulturen (z. B. Chilo suppressalis und Laodelphax).

In diesem Zusammenhang ist hervorzuheben, daß die genannten Verbindungen sich sowohl durch eine stark ausgeprägte systemische als auch Kontakt-Wirkung gegen saugende Insekten insbesondere gegen saugende Insekten der Ordnung Homoptera und vor allem gegen Insekten der Familie Aphididae (wie z. B. Aphis fabae, Aphis craccivora und Myzus persicae), welche sich mit bekannten Mitteln nur schwierig bekämpfen lassen, auszeichnen.

Wirkstoffe der Formel I zeigen auch eine sehr günstige Wirkung gegen Fliegen, wie z. B. Musca domestica und Mückenlarven und gegen ektoparasitäre Milben und Zecken z. B. der Familien Ixodidae, Argasidae und Dermanyssidae. Daneben zeichnen sich die Verbindungen der Formel I durch eine breite ovizide und ovolarvizide Wirkung aus.

Darüberhinaus besitzen die Verbindungen der Formel I fungizide und pflanzenregulatorische Eigenschaften und eine wertvolle Wirkung gegen pflanzenparasitäre Nematoden.

Die Verbindungen der Formel I werden in unveränderter Form oder vorzugsweise zusammen mit den in der Formulierungstechnik üblichen Hilfsmitteln eingesetzt und werden daher z. B. zu Emulionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in

bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $c_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnußöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien z. B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$—$C_{22}$), wie z. B. die Na- oder K-Salze der Öl- oder Stearinsäure, oder von natürlichen Fettsäuregemischen, die z. B. aus Kokosnuß- oder Tallöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8—22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4—14)-Äthylenoxid-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxid-Addukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglycoläther, Polypropylen-Polyäthylenoxid-Addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u. a. in folgender Publikation beschrieben:

»Mc Cutcheon's Detergents and Emulsifiers Annual« MC Publishing Corp., Ridgewood, New Jersey, 1979.

Die pestiziden Zubereitungen enthalten in der Regel 0,1 bis 99%, insbesondere 0,1 bis 95%, Wirkstoff der Formel I, 1 bis 99,9% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezielle Effekte enthalten.

Durch inniges Vermischen der Trägerstoffe mit dem Wirkstoff erhält man gebrauchsfertige Stäubemittel.

Formulierungsbeispiele für Wirkstoffe der Formel I (% = Gewichtsprozent)

1. Spritzpulver

| | a) | b) | c) |
|---|---|---|---|
| Wirkstoff | 25% | 50% | 75% |
| Na-Ligninsulfonat | 5% | 5% | – |
| Na-Laurylsulfat | 3% | – | 5% |
| Na-Diisobutylnaphthalinsulfonat | – | 6% | 10% |
| Octylphenolpolyäthylenglykoläther (7–8 Mol AeO) | – | 2% | – |
| Hochdisperse Kieselsäure | 5% | 10% | 10% |
| Kaolin | 62% | 27% | – |

Der Wirktoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2. Emulsion-Konzentrat

| | |
|---|---|
| Wirkstoff | 10% |
| Octylphenolpolyäthylenglykoläther (4–5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

3. Stäubemittel

| | a) | b) |
|---|---|---|
| Wirkstoff | 5% | 8% |
| Talkum | 95% | – |
| Kaolin | – | 92% |

Man erhält anwendungsfertige Stäubemittel, indem der Wirkstoff mit dem Träger vermischt und auf einer geeigneten Mühle vermahlen wird.

4. Extruder Granulat

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschließend im Luftstrom getrocknet.

5. Umhüllungs-Granulat

| | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmäßig aufgetragen. Auf diese Weise erhält man staubfreie Umhüllungs-Granulate.

6. Suspensions-Konzentrat

| | |
|---|---|
| Wirkstoff | 40% |
| Äthylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wäßrige Formaldehyd-Lösung | 0,2% |
| Silikonöl in Form einer 75%igen wäßrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1

Herstellung der Verbindung Nr. 1 der Formel

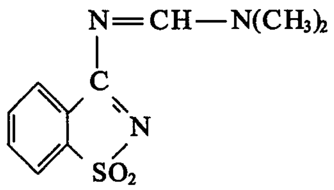

2,6 g 3-Amino-benzisothiazol-1,1-dioxid werden mit 7 ml Dimethylformamiddiäthylacetal vermischt und unter Rühren 5 Stunden bei 120°C gehalten. Nach dem Abkühlen wird das Produkt abgenutscht, auf der Nutsche mit Äther gewaschen und bei 20°C getrocknet. Man erhält die Verbindung Nr. 1 mit einem Schmelzpunkt von 233°C.

Auf analoge Weise werden auch folgende Verbindungen hergestellt:

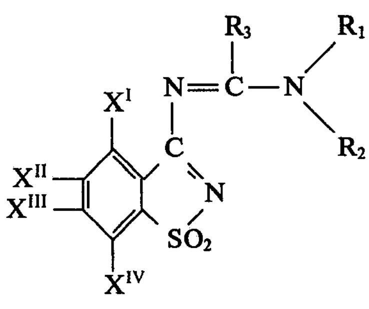

| Nr. | $R_1$ | $R_2$ | $R_3$ | $X^I$ | $X^{II}$ | $X^{III}$ | $X^{IV}$ | Physikalische Daten |
|---|---|---|---|---|---|---|---|---|
| 2 | $-C_4H_{9(n)}$ | $-C_4H_{9(n)}$ | H | H | H | H | H | Smp.: 109–110°C |
| 3 | $-CH_2-CH=CH_2$ | $-CH_2-CH=CH_2$ | H | H | H | H | H | Smp.: 133°C |
| 4 | $-CH_2-CH_2-CH_2-CH_2-CH_2-$ | $-CH_2-CH_2-CH_2-CH_2-CH_2-$ | H | H | H | H | H | Smp.: 225–227°C |
| 5 | $-CH_2-CH_2-O-CH_2-CH_2$ | $-CH_2-CH_2-O-CH_2-CH_2-$ | H | H | H | H | H | Smp.: 203°C |
| 6 | $-C_2H_5$ | $-C_2H_5$ | H | H | H | H | H | Harz |
| 7 | $-CH_3$ | $-CH_3$ | $-CH_3$ | H | H | H | H | Smp.: 170–175°C |
| 8 | $-CH_3$ | $-CH_3$ | H | Cl | H | H | H | Smp.: 240–245°C |
| 9 | $-CH_3$ | $-CH_3$ | $-CH_3$ | Cl | H | H | H | Smp.: 178–182°C |
| 10 | $-C_4H_{9(n)}$ | $-C_4H_{9(n)}$ | H | Cl | H | H | H | Smp.: 102–103°C |
| 11 | $-CH_2-CH_2-CH_2-CH_2-CH_2-$ | $-CH_2-CH_2-CH_2-CH_2-CH_2-$ | H | Cl | H | H | H | Smp.: 215–217°C |
| 12 | $-CH_2-CH_2-O-CH_2-CH_2-$ | $-CH_2-CH_2-O-CH_2-CH_2-$ | H | Cl | H | H | H | Smp.: 228–230°C |
| 13 | $-CH_2-CH=CH_2$ | $-CH_2-CH=CH_2$ | H | Cl | H | H | H | Smp.: 157–158°C |

## Beispiel 2

### Insektizide Kontakt-Wirkung: Aphis craccivora

In Töpfen angezogene Pflanzen (Vicia faba) werden vor dem Versuchsbeginn je mit ca. 200 Individuen der Spezies Aphis craccivora besiedelt. Die so behandelten Pflanzen werden 24 Stunden später mit einer Lösung enthaltend 200 oder 100 ppm der zu prüfenden Verbindung bis zur Tropfnässe besprüht. Man vewendet pro Test-Verbindung und pro Konzentration zwei Pflanzen, und eine Auswertung der erzielten Abtötungsrate erfolgt nach weiteren 24 Stunden.

Die Verbindungen gemäß Beispiel 1 zeigen im obigen Versuch die in der folgenden Tabelle angegebene Wirkung gegen Insekten der Spezies Aphis craccivora.

## Beispiel 3

### Systemisch-insektizide Wirkung: Aphis craccivora

Bewurzelte Bohnenpflanzen werden in Töpfe, welche 600 ccm Erde enthalten, verpflanzt. Anschließend werden 50 ml einer Lösung der zu prüfenden Verbindung (erhalten aus einem 25%igen Spritzpulver) in einer Konzentration von 50 ppm oder 12,5 ppm direkt auf die Erde aufgegossen.

Nach 24 Stunden werden auf die oberirdischen Pflanzenteile Läuse der Spezies Aphis craccivora gesetzt und die Pflanzen mit einem Plastikzylinder überstülpt, um die Läuse vor einer eventuellen Kontakt- oder Gaswirkung der Testsubstanz zu schützen.

Die Auswertung der erzielten Abtötung erfolgt 48 und 72 Stunden nach Versuchsbeginn. Pro Konzentrationsdosis Testsubstanz werden zwei Pflanzen, je eine in einem separaten Topf, verwendet. Der Versuch wird bei 25°C und 70% relativer Luftfeuchtigkeit durchgeführt.

Die Verbindungen gemäß Beispiel 1 zeigen im obigen Versuch die in der folgenden Tabelle angegebene systemische Wirkung gegen Insekten der Spezies Aphis craccivora.

### Biologische Versuchsergebnisse

In der folgenden Tabelle sind Versuchsergebnisse auf der Basis der vorstehenden Beispiele aufgeführt, und zwar mit folgendem Bewertungsindex in bezug auf die prozentuale Abtötung der Schädlinge.

A: 70–100% Abtötung bei 12,5 ppm Wirkstoffkonzentration
B: 70–100% Abtötung bei 50 ppm Wirkstoffkonzentration
C: 70–100% Abtötung bei 100 ppm Wirkstoffkonzentration
D: 70–100% Abtötung bei 200 ppm Wirkstoffkonzentration

| Verbindungen Nr. | Kontakt-Wirkung gegen Aphis craccivora | Systemische Wirkungen gegen Aphis craccivora |
|---|---|---|
| 1 | C | A |
| 2 | C | A |
| 3 | C | A |
| 4 | D | B |
| 5 | D | B |
| 6 | D | B |
| 7 | D | B |
| 8 | C | A |
| 9 | D | A |

Fortsetzung

| Verbindungen Nr. | Kontakt-Wirkung gegen Aphis craccivora | Systemische Wirkungen gegen Aphis craccivora |
|---|---|---|
| 10 | C | A |
| 11 | C | B |
| 12 | C | B |
| 13 | C | A |

**Patentansprüche für die Vertragsstaaten: BE, CH, DE, FR, IT, LI, NL**

1. 3-Amidino-benzisothiazol-1,1-dioxide der Formel

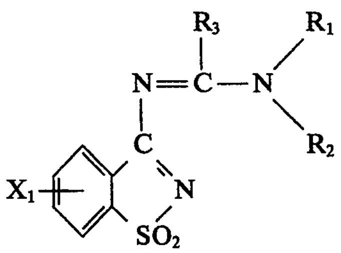

(I)

worin

$R_1$ und $R_2$ je $C_1$—$C_5$-Alkyl oder $C_2$—$C_5$-Alkenyl,
$R_3$ Wasserstoff oder Methyl oder
$R_1$ und $R_2$ zusammen eine gegebenenfalls durch O, S oder NH unterbrochene $C_3$—$C_6$-Alkylenkette oder
$R_1$ und $R_3$ zusammen $-CH_2-CH_2-CH_2-$ und
$X_1$ Wasserstoff, Halogen $C_1$—$C_5$-Alkyl, Halogen-$C_1$—$C_5$-alkyl oder $C_1$—$C_5$-Alkoxy bedeuten.

2. Eine Verbindung gemäß Anspruch 1, worin

$R_1$ und $R_2$ je $C_1$—$C_5$-Alkyl oder $C_2$—$C_5$-Alkenyl,
$R_3$ Wasserstoff oder Methyl oder
$R_1$ und $R_2$ zusammen eine gegebenenfalls durch O, S oder NH unterbrochene $C_3$—$C_6$-Alkylenkette oder
$R_1$ und $R_3$ zusammen $-CH_2-CH_2-CH_2-$ und
$X_1$ Wasserstoff, Halogen, $C_1$—$C_5$-Alkyl oder $C_1$—$C_5$-Alkoxy bedeuten.

3. Eine Verbindung gemäß Anspruch 1, worin $R_1$ und $R_2$ je $C_1$—$C_4$-Alkyl oder 2-Propenyl oder $R_1$ und $R_2$ zusammen

$$-CH_2-CH_2-CH_2-CH_2-CH_2-$$
$$-CH_2-CH_2-O-CH_2-CH_2-$$
$$-CH_2-CH_2-S-CH_2-CH_2-$$

oder

$$-CH_2-CH_2-NH-CH_2-CH_2-$$

$R_3$ Wasserstoff und $X_1$ Wasserstoff, Halogen, Methyl, Trifluormethyl oder Methoxy bedeuten.

4. Eine Verbindung gemäß Anspruch 3, worin $R_1$ und $R_2$ je $C_1$—$C_4$-Alkyl oder 2-Propenyl oder $R_1$ und $R_2$ zusammen

$—CH_2—CH_2—CH_2—CH_2—CH_2—$

$—CH_2—CH_2—O—CH_2—CH_2—$

$—CH_2—CH_2—S—CH_2—CH_2—$

oder

$—CH_2—CH_2—NH—CH_2—CH_2—$

$R_3$ Wasserstoff und $X_1$ Wasserstoff, Chlor, Methyl oder Methoxy bedeuten.

5. Eine Verbindung gemäß Anspruch 4, worin $R_1$ und $R_2$ je Methyl, n-Butyl oder 2-Propenyl oder $R_1$ und $R_2$ zusammen

$—CH_2—CH_2—CH_2—CH_2—CH_2—$

oder

$—CH_2—CH_2—O—CH_2—CH_2—$

$R_3$ Wasserstoff und $X_1$ Wasserstoff oder Chlor bedeuten.

6. Eine Verbindung gemäß Anspruch 5, worin $R_1$ und $R_2$ je Methyl, n-Butyl oder 2-Propenyl oder $R_1$ und $R_2$ zusammen

$—CH_2—CH_2—CH_2—CH_2—CH_2—$

oder

$—CH_2—CH_2—O—CH_2—CH_2—$

$R_3$ und $X_1$ je Wasserstoff bedeuten.

7. Die Verbindung gemäß Anspruch 6 der Formel

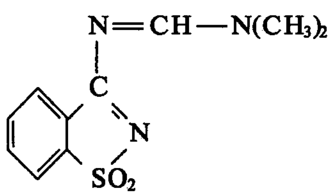

8. Die Verbindung gemäß Anspruch 6 der Formel

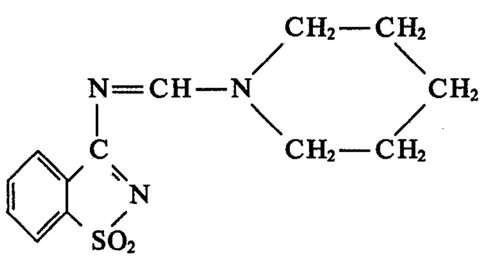

9. Die Verbindung gemäß Anspruch 6 der Formel

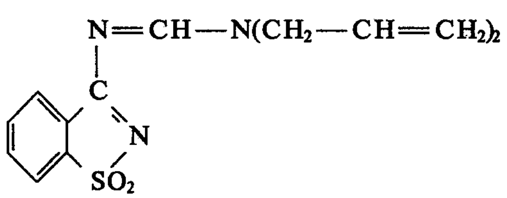

10. Die Verbindung gemäß Anspruch 6 der Formel

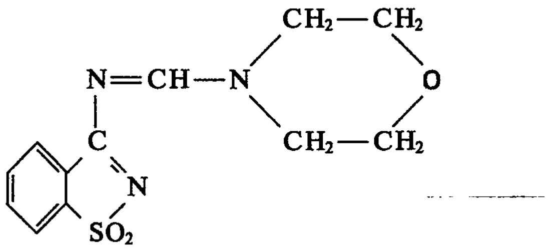

11. Die Verbindung gemäß Anspruch 6 der Formel

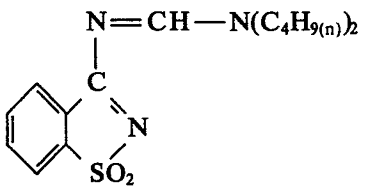

12. Die Verbindung gemäß Anspruch 2 der Formel

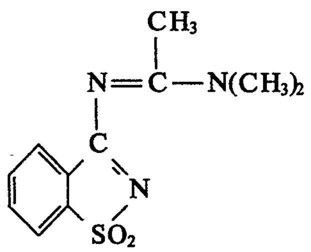

13. Die Verbindung gemäß Anspruch 5 der Formel

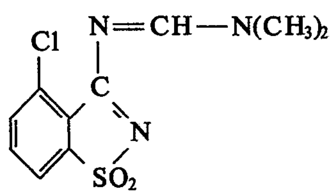

14. Die Verbindung gemäß Anspruch 5 der Formel

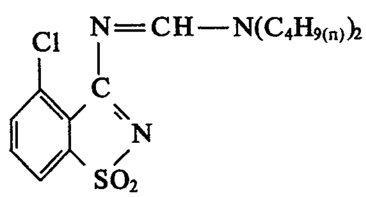

15. Die Verbindung gemäß Anspruch 5 der Formel

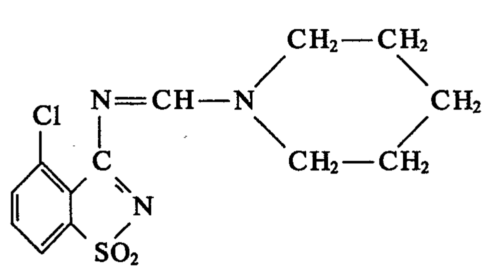

16. Die Verbindung gemäß Anspruch 5 der Formel

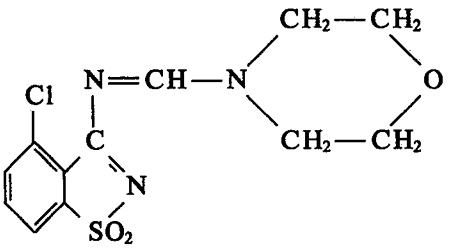

17. Die Verbindung gemäß Anspruch 5 der Formel

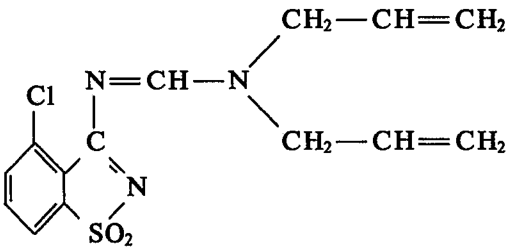

18. Die Verbindung gemäß Anspruch 2 der Formel

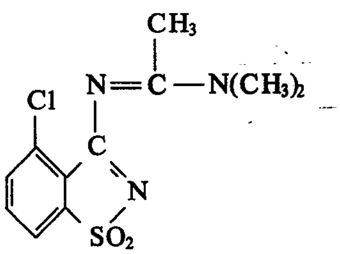

19. Verfahren zur Herstellung von Verbindungen gemäß Anspruch 1, dadurch gekennzeichnet, daß man eine Verbindung der Formel

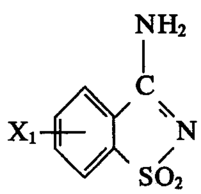

mit einer Verbindung der Formel

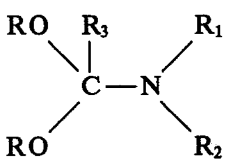

umsetzt, worin $R_1$, $R_2$, $R_3$ und $X_1$ die im Anspruch 1 angegebene Bedeutung haben und R für $C_1$—$C_5$-Alkyl steht.

20. Ein Schädlingsbekämpfungsmittel, welches als aktive Komponente eine Verbindung gemäß Anspruch 1 enthält.

21. Die Verwendung einer Verbindung gemäß Anspruch 1 zur Bekämpfung von Schädlingen an Tieren und Pflanzen.

22. Die Verwendung gemäß Anspruch 21 zur Bekämpfung von Insekten und Vertretern der Ordnung Akarina.

**Patentansprüche für den Vertragsstaat: AT**

1. Ein Schädlingsbekämpfungsmittel, welches neben flüssigen und festen Zusatzstoffen als aktive Komponente ein 3-Amidino-benzisothiazol-1,1-dioxid der Formel

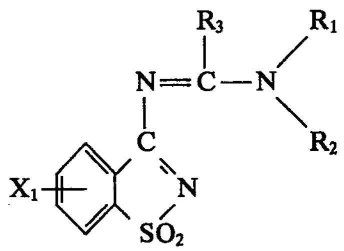

(I)

enthält, worin

$R_1$ und $R_2$ je $C_1$—$C_5$-Alkyl oder $C_2$—$C_5$-Alkenyl,
$R_3$ Wasserstoff oder Methyl oder
$R_1$ und $R_2$ zusammen eine gegebenenfalls durch O, S oder NH unterbrochene $C_3$—$C_6$-Alkylenkette oder
$R_1$ und $R_3$ zusammen $-CH_2-CH_2-CH_2-$ und
$X_1$ Wasserstoff, Halogen, $C_1$—$C_5$-Alkyl, Halogen-$C_1$—$C_5$-alkyl oder $C_1$—$C_5$-Alkoxy bedeuten.

2. Ein Mittel gemäß Anspruch 1, worin in der Formel I

$R_1$ und $R_2$ je $C_1$—$C_5$-Alkyl oder $C_2$—$C_5$-Alkenyl,
$R_3$ Wasserstoff oder Methyl oder
$R_1$ und $R_2$ zusammen eine gegebenenfalls durch O, S oder NH unterbrochene $C_3$—$C_6$-Alkylenkette oder
$R_1$ und $R_3$ zusammen $-CH_2-CH_2-CH_2-$ und
$X_1$ Wasserstoff, Halogen, $C_1$—$C_5$-Alkyl oder $C_1$—$C_5$-Alkoxy bedeuten.

3. Ein Mittel gemäß Anspruch 1, worin in der Formel I $R_1$ und $R_2$ je $C_1$—$C_4$-Alkyl oder 2-Propenyl oder $R_1$ und $R_2$ zusammen

$-CH_2-CH_2-CH_2-CH_2-CH_2-$

$-CH_2-CH_2-O-CH_2-CH_2-$

$-CH_2-CH_2-S-CH_2-CH_2-$

oder

$-CH_2-CH_2-NH-CH_2-CH_2-$

$R_3$ Wasserstoff und $X_1$ Wasserstoff, Halogen, Methyl, Trifluormethyl oder Methoxy bedeuten.

4. Ein Mittel gemäß Anspruch 3, worin in der Formel I $R_1$ und $R_2$ je $C_1$—$C_4$-Alkyl oder 2-Propenyl oder $R_1$ und $R_2$ zusammen

$-CH_2-CH_2-CH_2-CH_2-CH_2-$

$-CH_2-CH_2-O-CH_2-CH_2-$

$-CH_2-CH_2-S-CH_2-CH_2-$

oder

$-CH_2-CH_2-NH-CH_2-CH_2-$

$R_3$ Wasserstoff und $X_1$ Wasserstoff, Chlor, Methyl oder Methoxy bedeuten.

5. Ein Mittel gemäß Anspruch 4, worin in der Formel I $R_1$ und $R_2$ je Methyl, n-Butyl oder 2-Propenyl oder $R_1$ und $R_2$ zusammen

$-CH_2-CH_2-CH_2-CH_2-CH_2-$

oder

$$—CH_2—CH_2—O—CH_2—CH_2—$$

$R_3$ Wasserstoff und $X_1$ Wasserstoff oder Chlor bedeuten.

6. Ein Mittel gemäß Anspruch 5, worin in der Formel I $R_1$ und $R_2$ je Methyl, n-Butyl oder 2-Propenyl oder $R_1$ und $R_2$ zusammen

$$—CH_2—CH_2—CH_2—CH_2—CH_2—$$

oder

$$—CH_2—CH_2—O—CH_2—CH_2—$$

$R_3$ und $X_1$ je Wasserstoff bedeuten.

7. Ein Mittel gemäß Anspruch 6, welches als aktive Komponente die Verbindung der Formel

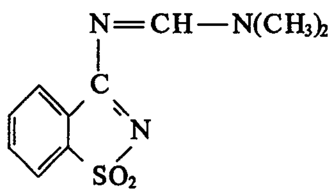

enthält.

8. Ein Mittel gemäß Anspruch 6, welches als aktive Komponente die Verbindung der Formel

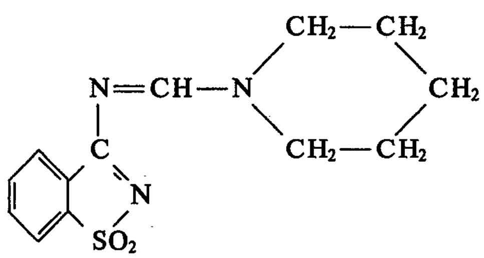

enthält.

9. Ein Mittel gemäß Anspruch 6, welches als aktive Komponente die Verbindung der Formel

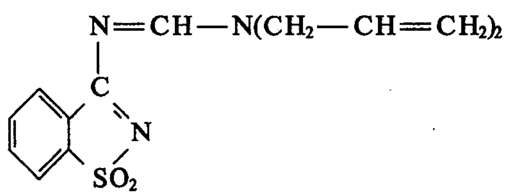

enthält.

10. Ein Mittel gemäß Anspruch 6, welches als aktive Komponente die Verbindung der Formel

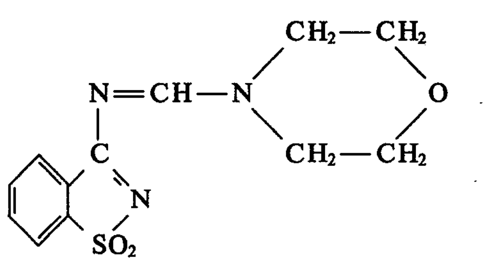

enthält.

11. Ein Mittel gemäß Anspruch 6, welches als aktive Komponente die Verbindung der Formel

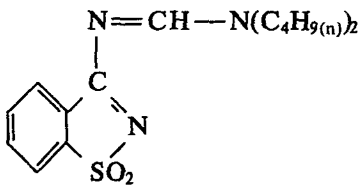

enthält.

12. Ein Mittel gemäß Anspruch 2, welches als aktive Komponente die Verbindung der Formel

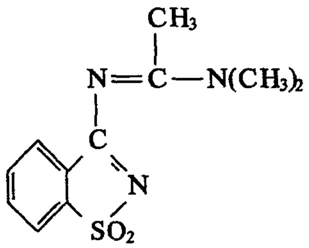

enthält.

13. Ein Mittel gemäß Anspruch 5, welches als aktive Komponente die Verbindung der Formel

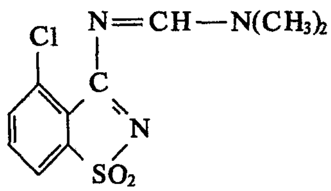

enthält.

14. Ein Mittel gemäß Anspruch 5, welches als aktive Komponente die Verbindung der Formel

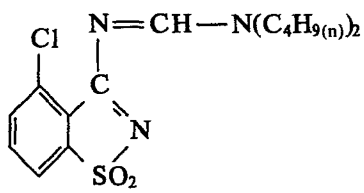

enthält.

15. Ein Mittel gemäß Anspruch 5, welches als aktive Komponente die Verbindung der Formel

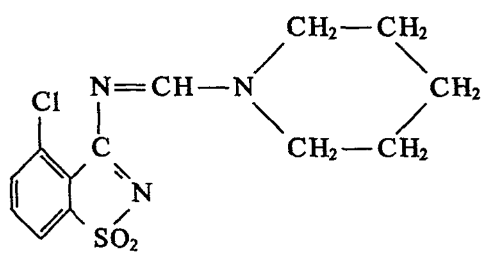

enthält.

16. Ein Mittel gemäß Anspruch 5, welches als aktive Komponente die Verbindung der Formel

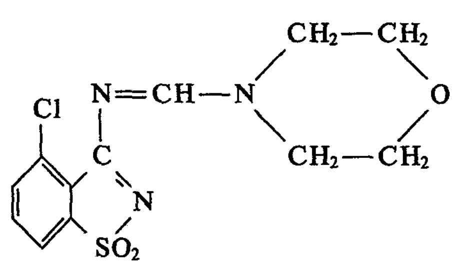

enthält.

17. Ein Mittel gemäß Anspruch 5, welches als aktive Komponente die Verbindung der Formel

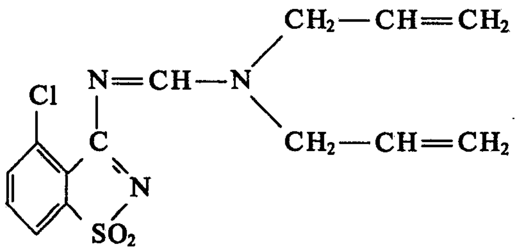

enthält.

18. Ein Mittel gemäß Anspruch 2, welches als aktive Komponente die Verbindung der Formel

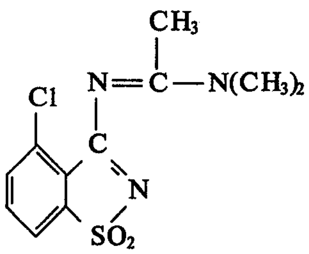

enthält.

19. Verfahren zur Herstellung von 3-Amidino-benzisothiazol-1,1-dioxiden der Formel

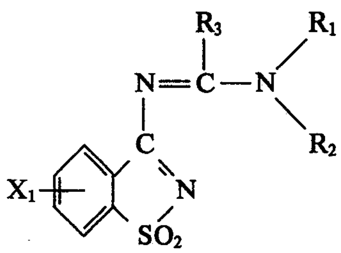

(I)

enthält, worin

| | |
|---|---|
| $R_1$ und $R_2$ | je $C_1$—$C_5$-Alkyl oder $C_2$—$C_5$-Alkenyl, |
| $R_3$ | Wasserstoff oder Methyl oder |
| $R_1$ und $R_2$ | zusammen eine gegebenenfalls durch O, S oder NH unterbrochene $C_3$—$C_6$-Alkylenkette oder |
| $R_1$ und $R_3$ | zusammen $-CH_2-CH_2-CH_2-$ und |
| $X_1$ | Wasserstoff, Halogen, $C_1$—$C_5$-Alkyl, Halogen-$C_1$—$C_5$-alkyl oder $C_1$—$C_5$-Alkoxy bedeuten, |

dadurch gekennzeichnet, daß man eine Verbindung der Formel

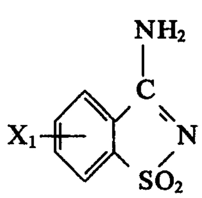

mit einer Verbindung der Formel

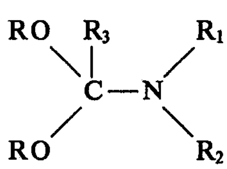

umsetzt, worin $R_1$, $R_2$, $R_3$ und $X_1$ die oben angegebene Bedeutung haben und R für $C_1$—$C_5$-Alkyl steht.

20. Die Verwendung eines Mittels gemäß Anspruch 1 zur Bekämpfung von Schädlingen an Tieren und Pflanzen.

21. Die Verwendung gemäß Anspruch 21 zur Bekämpfung von Insekten und Vertretern der Ordnung Akarina.

**Claims for the Contracting States: BE, DE, FR, IT, NL, CH, LI**

1. 3-Amidinobenzisothiazole-1,1-dioxide of the formula I

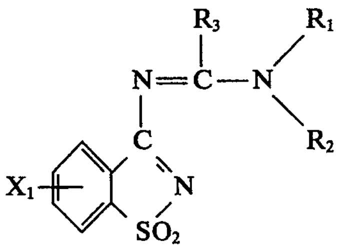

(I)

wherein

$R_1$ and $R_2$ are each $C_1$—$C_5$alkyl or $C_2$—$C_5$alkenyl,
$R_3$ is hydrogen or methyl, or
$R_1$ and $R_2$ together form a $C_3$—$C_6$alkylene chain which can be interrupted by O, S or NH, or
$R_1$ and $R_3$ together are $-CH_2-CH_2-CH_2-$, and
$X_1$ is hydrogen, halogen, $C_1$—$C_5$alkyl, $C_1$—$C_5$haloalkyl or $C_1$—$C_5$alkoxy.

2. A compound according to claim 1, wherein

$R_1$ and $R_2$ are each $C_1$—$C_5$alkyl or $C_2$—$C_5$alkenyl,
$R_3$ is hydrogen or methyl, or
$R_1$ and $R_2$ together form a $C_3$—$C_6$alkylene chain which can be interrupted by O, S or NH, or
$R_1$ and $R_3$ together are $-CH_2-CH_2-CH_2-$, and
$X_1$ is hydrogen, halogen, $C_1$—$C_5$alkyl or $C_1$—$C_5$alkoxy.

3. A compound according to claim 1, wherein $R_1$ and $R_2$ are each $C_1$—$C_4$alkyl or 2-propenyl, or $R_1$ and $R_2$ together are

$$-CH_2-CH_2-CH_2-CH_2-CH_2-$$
$$-CH_2-CH_2-O-CH_2-CH_2-$$
$$-CH_2-CH_2-S-CH_2-CH_2-$$

or

$$-CH_2-CH_2-NH-CH_2-CH_2-$$

$R_3$ is hydrogen, and $X_1$ is hydrogen, halogen, methyl, trifluoromethyl or methoxy.

4. A compound according to claim 3, wherein $R_1$ and $R_2$ are each $C_1$—$C_4$alkyl or 2-propenyl, or $R_1$ and $R_2$ together are

$$-CH_2-CH_2-CH_2-CH_2-CH_2-$$
$$-CH_2-CH_2-O-CH_2-CH_2-$$
$$-CH_2-CH_2-S-CH_2-CH_2-$$

or

$$-CH_2-CH_2-NH-CH_2-CH_2-$$

$R_3$ is hydrogen, and $X_1$ is hydrogen, chlorine, methyl or methoxy.

5. A compound according to claim 4, wherein $R_1$ and $R_2$ are each methyl, n-butyl or 2-propenyl, or $R_1$ and $R_2$ together are

$—CH_2—CH_2—CH_2—CH_2—CH_2—$

or

$—CH_2—CH_2—O—CH_2—CH_2—$

$R_3$ is hydrogen, and $X_1$ is hydrogen or chlorine.

6. A compound according to claim 5, wherein $R_1$ and $R_2$ are each methyl, n-butyl or 2-propenyl, or $R_1$ and $R_2$ together are

$—CH_2—CH_2—CH_2—CH_2—CH_2—$

or

$—CH_2—CH_2—O—CH_2—CH_2—$

and $R_3$ and $X_1$ are each hydrogen.

7. The compound according to claim 6 of the formula

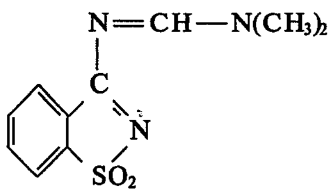

8. The compound according to claim 6 of the formula

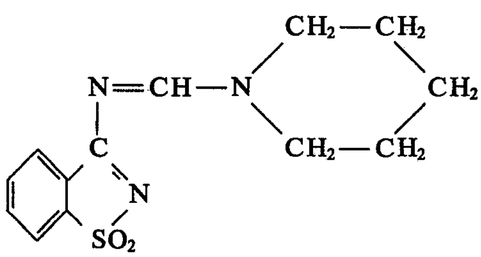

9. The compound according to claim 6 of the formula

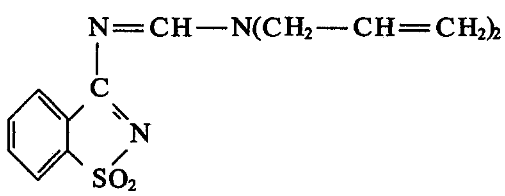

10. The compound according to claim 6 of the formula

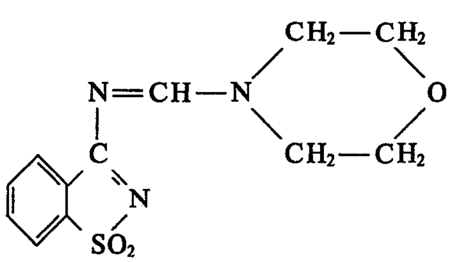

11. The compound according to claim 6 of the formula

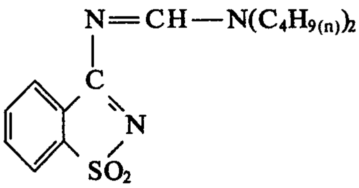

12. The compound according to claim 2 of the formula

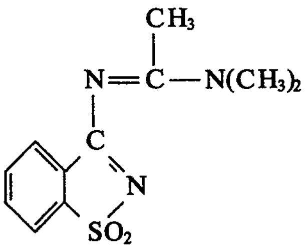

13. The compound according to claim 5 of the formula

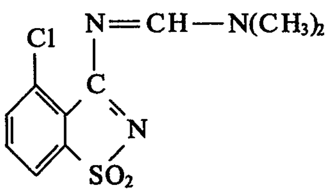

14. The compound according to claim 5 of the formula

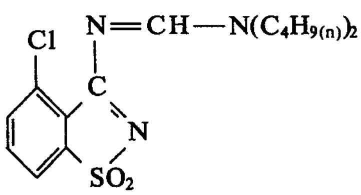

15. The compound according to claim 5 of the formula

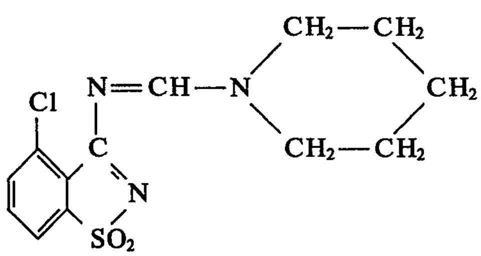

16. The compound according to claim 5 of the formula

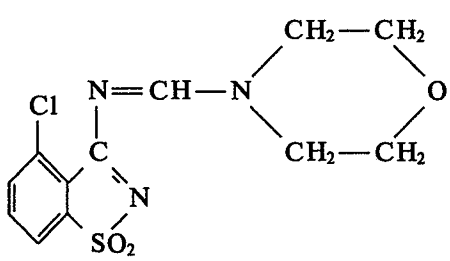

17. compound according to claim 5 of the formula

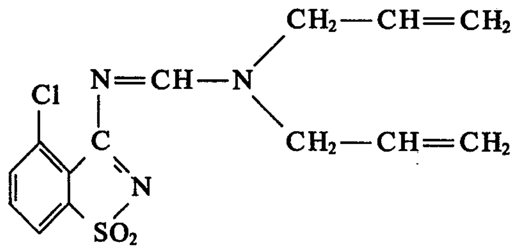

18. The compound according to claim 2 of the formula

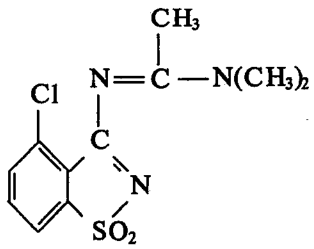

19. A process for the preparation of a compound according to claim 1, which process comprises reacting a compound of the formula

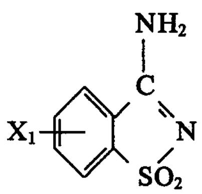

with a compound of the formula

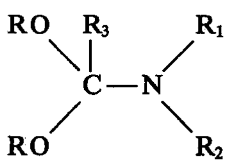

wherein $R_1$, $R_2$, $R_3$ and $X_1$ are as defined in claim 1, and R is $C_1$—$C_5$alkyl.

20. A pesticidal composition which contains as active ingredient a compound according to claim 1.

21. A method of controlling pests of animals and plants, which method comprises applying thereto or to the locus thereof an effective amount of a compound according to claim 1.

22. A method according to claim 21 for controlling insects and members of the order Acarina.

**Claims for the Contracting State: AT**

1. A pesticidal composition which contains as active ingredient a 3-amidinobenzisothiazole-1,1-dioxide of the formula

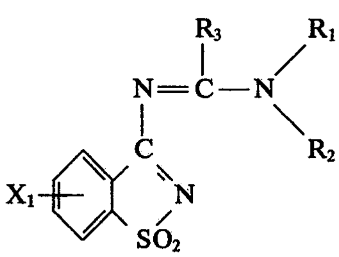

(I)

wherein

$R_1$ and $R_2$ are each $C_1$—$C_5$alkyl or $C_2$—$C_5$alkenyl,

$R_3$ is hydrogen or methyl, or
$R_1$ and $R_2$ together form a $C_3$–$C_6$alkylene chain with can be interrupted by O, S or NH, or
$R_1$ and $R_3$ together are $-CH_2-CH_2-CH_2-$, and
$X_1$ is hydrogen, halogen, $C_1$–$C_5$alkyl, $C_1$–$C_5$haloalkyl or $C_1$–$C_5$alkoxy,

together with liquid and solid adjuvants.

2. A composition according to claim 1, wherein in formula I

$R_1$ and $R_2$ are each $C_1$–$C_5$alkyl or $C_2$–$C_5$alkenyl,
$R_3$ is hydrogen or methyl, or
$R_1$ and $R_2$ together form a $C_3$–$C_6$alkylene chain which can be interrupted by O, S or NH, or
$R_1$ and $R_3$ together are $-CH_2-CH_2-CH_2-$, and
$X_1$ is hydrogen, halogen, $C_1$–$C_5$alkyl or $C_1$–$C_5$alkoxy.

3. A composition according to claim 1, wherein in formula I $R_1$ and $R_2$ are each $C_1$–$C_4$alkyl or 2-propenyl, or $R_1$ and $R_2$ together are

$$-CH_2-CH_2-CH_2-CH_2-CH_2-$$
$$-CH_2-CH_2-O-CH_2-CH_2-$$
$$-CH_2-CH_2-S-CH_2-CH_2-$$

or

$$-CH_2-CH_2-NH-CH_2-CH_2-$$

$R_3$ is hydrogen, and $X_1$ is hydrogen, halogen, methyl, trifluoromethyl or methoxy.

4. A composition according to claim 3, wherein in formula I $R_1$ and $R_2$ are each $C_1$–$C_4$alkyl or 2-propenyl, or $R_1$ and $R_2$ togethe are

$$-CH_2-CH_2-CH_2-CH_2-CH_2-$$
$$-CH_2-CH_2-O-CH_2-CH_2-$$
$$-CH_2-CH_2-S-CH_2-CH_2-$$

or

$$-CH_2-CH_2-NH-CH_2-CH_2-$$

$R_3$ is hydrogen, and $X_1$ is hydrogen, chlorine, methyl or methoxy.

5. A composition according to claim 4, wherein in formula I $R_1$ and $R_2$ are each methyl, n-butyl or 2-propenyl, or $R_1$ and $R_2$ together are

$$-CH_2-CH_2-CH_2-CH_2-CH_2-$$

or

$$-CH_2-CH_2-O-CH_2-CH_2-$$

$R_3$ is hydrogen, and $X_1$ is hydrogen or chlorine.

6. A composition according to claim 5, wherein in formula I $R_1$ and $R_2$ are each methyl, n-butyl or 2-propenyl, or $R_1$ and $R_2$ together are

$$-CH_2-CH_2-CH_2-CH_2-CH_2-$$

or

$$-CH_2-CH_2-O-CH_2-CH_3-$$

and $R_3$ and $X_1$ are each hydrogen.

7. A composition according to claim 6, which contains as active ingredient the compound of the formula

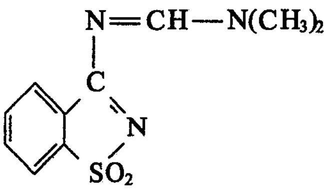

8. A composition according to claim 6, which contains as active ingredient the compound of the formula

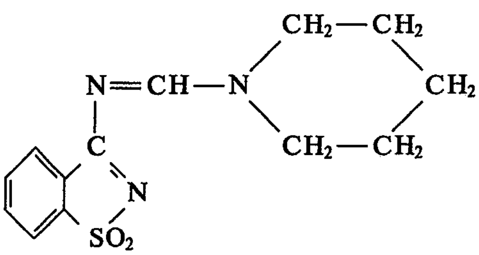

9. A composition according to claim 6, which contains as active ingredient the compound of the formula

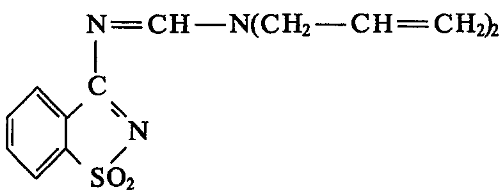

10. A composition according to claim 6, which contains as active ingredient the compound of the formula

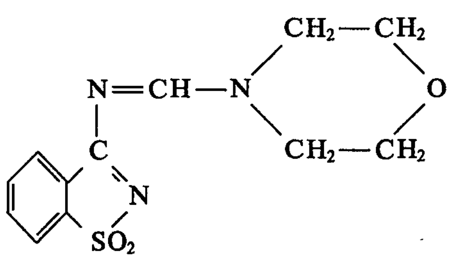

11. A composition according to claim 6, which contains as active ingredient the compound of the formula

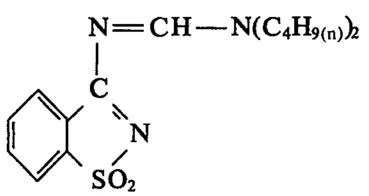

12. A composition according to claim 2, which contains as active ingredient the compound of the formula

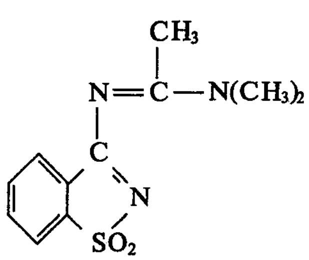

13. A composition according to claim 5, which contains as active ingredient the compound of the formula

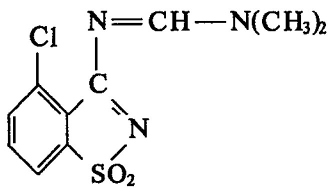

14. A composition according to claim 5, which contains as active ingredient the compound of the formula

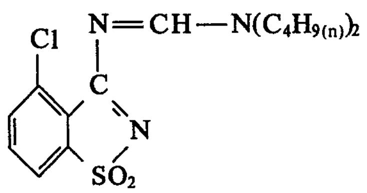

15. A composition according to claim 5, which contains as active ingredient the compound of the formula

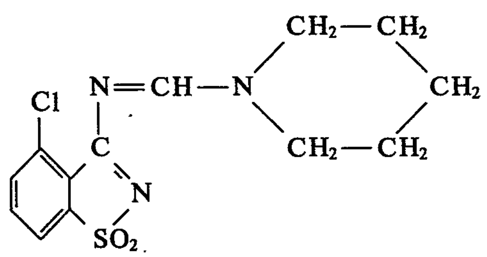

16. A composition according to claim 5, which contains as active ingredient the compound of the formula

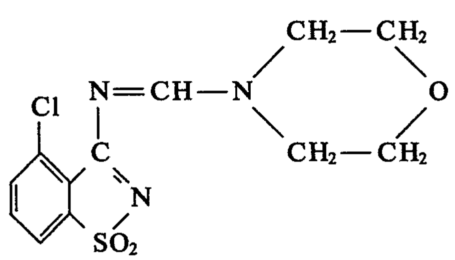

17. A composition according to claim 5, which contains as active ingredient the compound of the formula

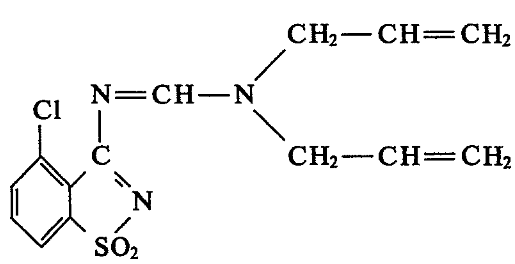

18. A composition according to claim 2, which contains as active ingredient the compound of the formula

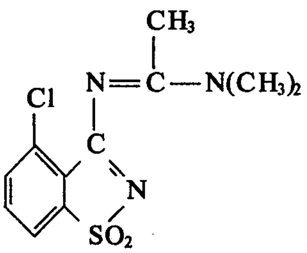

19. A process for the preparation of a 3-amidinobenzisothiazole-1,1-dioxide of the formula

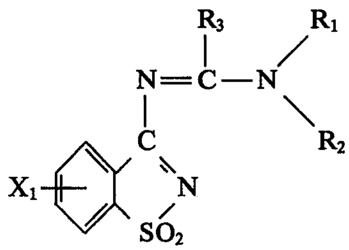

(I)

wherein

| | |
|---|---|
| $R_1$ and $R_2$ | are each $C_1$—$C_5$alkyl or $C_2$—$C_5$alkenyl, |
| $R_3$ | is hydrogen or methyl, or |
| $R_1$ and $R_2$ | together form a $C_3$—$C_6$alkylene chain which can be interrupted by O, S or NH, or |
| $R_1$ and $R_3$ | together are $-CH_2-CH_2-CH_2-$, and |
| $X_1$ | is hydrogen, halogen, $C_1$—$C_5$alkyl, $C_1$—$C_5$haloalkyl or $C_1$—$C_5$alkoxy, |

which process comprises reacting a compound of the formula

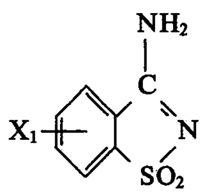

with a compound of the formula

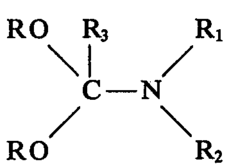

wherein $R_1$, $R_2$, $R_3$ and $X_1$ are as defined above, and R is $C_1$—$C_5$alkyl.

20. A method of controlling pests of animals and plants, which method comprises applying thereto or to the locus thereof an effective amount of a composition according to claim 1.

21. A method according to claim 20 for controlling insects and members of the order Acarina.

**Revendications pour les Etats contractants: BE, DE, FR, IT, NL, CH, LI**

1. 3-amidino-benzisothiazol-1,1-dioxydes de formule

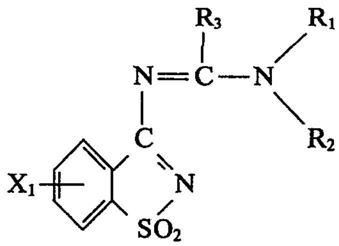

(I)

où

$R_1$ et $R_2$ représentent chacun un alcoyle en $C_1$ à $C_5$ ou un alcényle en $C_2$ à $C_5$,
$R_3$ représente un hydrogène ou un méthyle ou
$R_1$ et $R_2$ représentent ensemble une chaîne alcoylène en $C_3$ à $C_6$ éventuellement interrompue par O, S ou NH ou
$R_1$ et $R_3$ représentent ensemble $-CH_2-CH_2-CH_2-$ et
$X_1$ représente un hydrogène, un halogène, un alcoyle en $C_1$ à $C_5$, un halogène-alcoyle en $C_1$ à $C_5$ ou un alcoxy en $C_1$ à $C_5$.

2. Composé selon la revendication 1, où

$R_1$ et $R_2$ représentent chacun un alcoyle en $C_1$ à $C_5$ ou un alcényle en $C_2$ à $C_5$,
$R_3$ représente un hydrogène ou un méthyle ou
$R_1$ et $R_2$ représentent ensemble une chaîne alcoylène en $C_3$ à $C_6$ éventuellement interrompue par O, S ou NH ou
$R_1$ et $R_3$ représentent ensemble $-CH_2-CH_2-CH_2-$ et
$X_1$ représente un hydrogène, un halogène, un alcoyle en $C_1$ à $C_5$ ou un alcoxy en $C_1$ à $C_5$.

3. Composé selon la revendication 1, où $R_1$ et $R_2$ représentent chacun un alcoyle en $C_1$ à $C_4$ ou un 2-propényle ou $R_1$ et $R_2$ ensemble représentent

$-CH_2-CH_2-CH_2-CH_2-CH_2-$

$-CH_2-CH_2-O-CH_2-CH_2-$

$-CH_2-CH_2-S-CH_2-CH_2-$

ou

$-CH_2-CH_2-NH-CH_2-CH_2-$

$R_3$ représente un hydrogène et $X_1$ représente un hydrogène, un halogène, un méthyle, un trifluorométhyle ou un méthoxy.

4. Composé selon la revendication 3, où $R_1$ et $R_2$ représentent chacun un alcoyle en $C_1$ à $C_4$ ou un 2-propényle ou $R_1$ et $R_2$ représentent ensemble

$-CH_2-CH_2-CH_2-CH_2-CH_2-$

$-CH_2-CH_2-O-CH_2-CH_2-$

$-CH_2-CH_2-S-CH_2-CH_2-$

ou

$-CH_2-CH_2-NH-CH_2-CH_2-$

$R_3$ représente un hydrogène et $X_1$ représente un hydrigène, un chlore, un méthyle ou un méthoxy.

5. Composé selon la revendication 4, où $R_1$ et $R_2$ représentent chacun un méthyle, un n-butyle ou un 2-propényl ou $R_1$ et $R_2$ représentent ensemble

$-CH_2-CH_2-CH_2-CH_2-CH_2-$

ou

$-CH_2-CH_2-O-CH_2-CH_2-$

$R_3$ représente un hydrogène et $X_1$ représente un hydrogène ou un chlore.

6. Composé selon la revendication 5, où $R_1$ et $R_2$ représentent chacun un méthyle, un n-butyle ou un 2-propényle ou $R_1$ et $R_2$ représentent ensemble

$$-CH_2-CH_2-CH_2-CH_2-CH_2-$$

ou

$$-CH_2-CH_2-O-CH_2-CH_2-$$

$R_3$ et $X_1$ représentent chacun un hydrogène.

7. Le composé selon la revendication 6 de formule

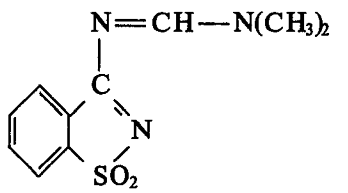

8. Le composé selon la revendication 6 de formule

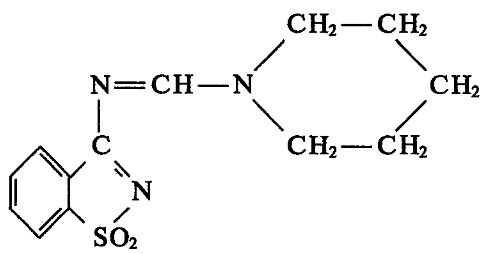

9. Le composé selon la revendication 6 de formule

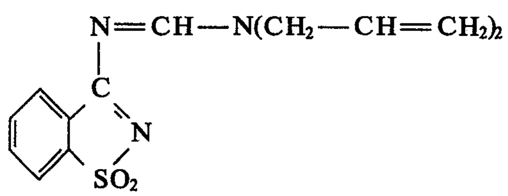

10. Le composé selon la revendication 6 de formule

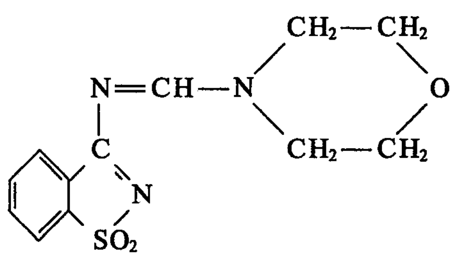

11. Le composé selon la revendication 6 de formule

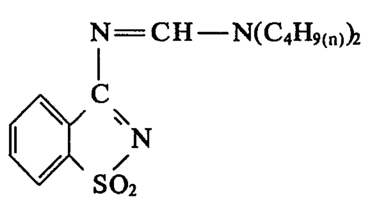

12. Le composé selon la revendication 2 de formule

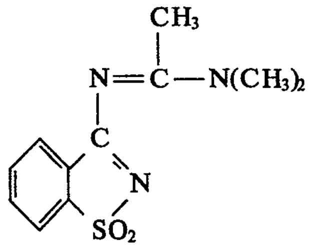

13. Le composé selon la revendication 5 de formule

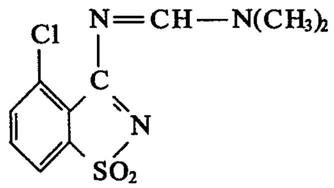

14. Le composé selon la revendication 5 de formule

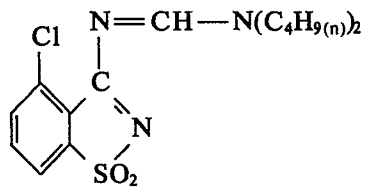

15. Le composé selon la revendication 5 de formule

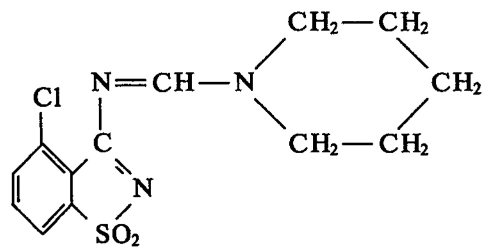

16. Le composé selon la revendication 5 de formule

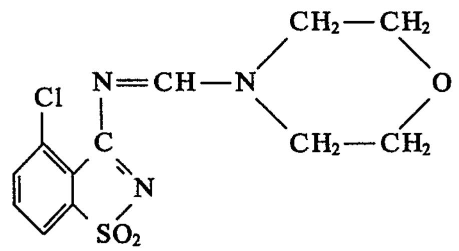

17. Le composé selon la revendication 5 de formule

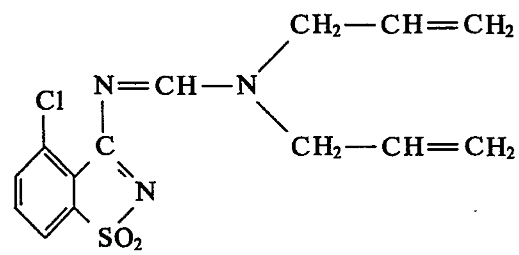

18. Le composé selon la revendication 2 de formule

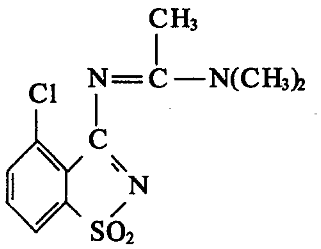

19. Procédé de préparation de composés selon la revendication 1, caractérisé en ce qu'on fait réagir un composé de formule

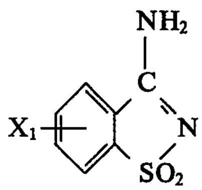

avec un composé de formule

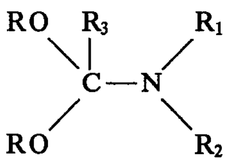

où $R_1$, $R_2$, $R_3$ et $X_1$ ont la signification donnée dans la revendication 1 et R représente un alcoyle en $C_1$ à $C_5$.

20. Agent de lutte antiparasitaire contenant comme composant actif un composé selon la revendication 1.

21. Application d'un composé selon la revendication 1 à la lutte contre les parasites des animaux et des végétaux.

22. Application selon la revendication 21 à la lutte contre les insectes et les représentants de l'ordre des acariens.

**Revendications pour l'Etat contractant: AT**

1. Agent de lutte antiparasitaire, contenant, outre des additifs liquides et solides comme composant actif un 3-amidino-benzisothiazol-1,1-dioxyde de formule

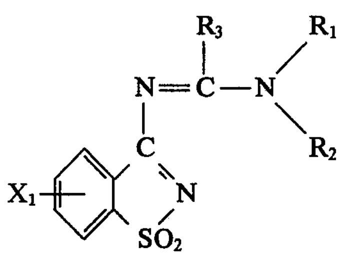

(I)

où

| | |
|---|---|
| $R_1$ et $R_2$ | représentent chacun un alcoyle en $C_1$ à $C_5$ ou un alcényle en $C_2$ à $C_5$, |
| $R_3$ | représente un hydrogène ou un méthyle ou |
| $R_1$ et $R_2$ | représentent ensemble une chaîne alcoylène en $C_3$ à $C_6$ éventuellement interrompue par O, S ou NH ou |
| $R_1$ et $R_3$ | représentent ensemble $-CH_2-CH_2-CH_2-$ et |
| $X_1$ | représente un hydrogène, un halogène, un alcoyle en $C_1$ à $C_5$, un halogène-alcoyle en $C_1$ à $C_5$ ou un alcoxy en $C_1$ à $C_5$. |

2. Agent selon la revendication 1, où dans la formule 1

$R_1$ et $R_2$ représentent chacun un alcoyle en $C_1$ à $C_5$ ou un alcényle en $C_2$ à $C_5$,
$R_3$ représente un hydrogène ou un méthyle ou
$R_1$ et $R_2$ représentent ensemble une chaîne alcoylène en $C_3$ à $C_6$ éventuellement interrompue par O, S ou NH ou
$R_1$ et $R_3$ représentent ensemble $-CH_2-CH_2-CH_2-$ et
$X_1$ représente un hydrogène, un halogène, un alcoyle en $C_1$ à $C_5$ ou un alcoxy en $C_1$ à $C_5$.

3. Agent selon la revendication 1, où dans la formule I $R_1$ et $R_2$ représentent chacun un alcoyle en $C_1$ à $C_4$ ou un 2-propényle ou $R_1$ et $R_2$ ensemble représentent

$-CH_2-CH_2-CH_2-CH_2-CH_2-$

$-CH_2-CH_2-O-CH_2-CH_2-$

$-CH_2-CH_2-S-CH_2-CH_2-$

ou

$-CH_2-CH_2-NH-CH_2-CH_2-$

$R_3$ représente un hydrogène et $X_1$ représente un hydrogène, un halogène, un méthyle, un trifluorométhyle ou un méthoxy.

4. Agent selon la revendication 3, où dans la formule I, $R_1$ et $R_2$ représentent chacun un alcoyle en $C_1$ à $C_4$ ou un 2-propényle ou $R_1$ et $R_2$ représentent ensemble

$-CH_2-CH_2-CH_2-CH_2-CH_2-$

$-CH_2-CH_2-O-CH_2-CH_2-$

$-CH_2-CH_2-S-CH_2-CH_2-$

ou

$-CH_2-CH_2-NH-CH_2-CH_2-$

$R_3$ représente un hydrogène et $X_1$ représente un hydrogène, un chlore, un méthyle ou un méthoxy.

5. Agent selon la revendication 4, où dans la formule I, $R_1$ et $R_2$ représentent chacun un méthyle, un n-butyle ou un 2-propényle ou $R_1$ et $R_2$ représentent ensemble

$-CH_2-CH_2-CH_2-CH_2-CH_2-$

ou

$-CH_2-CH_2-O-CH_2-CH_2-$

$R_3$ représente un hydrogène et $X_1$ représente un hydrogène ou un chlore.

6. Agent selon la revendication 5, où dans la formule I, $R_1$ et $R_2$ représentent chacun un méthyle, un n-butyle ou un 2-propényle ou $R_1$ et $R_2$ représentent ensemble

$-CH_2-CH_2-CH_2-CH_2-CH_2-$

ou

$-CH_2-CH_2-O-CH_2-CH_2-$

$R_3$ et $X_1$ représentent chacun un hydrogène.

7. Agent selon la revendication 6, qui contient comme composant actif le composé de formule

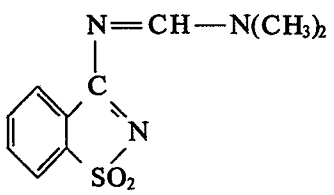

8. Agent selon la revendication 6 qui contient comme composant actif le composé de formule

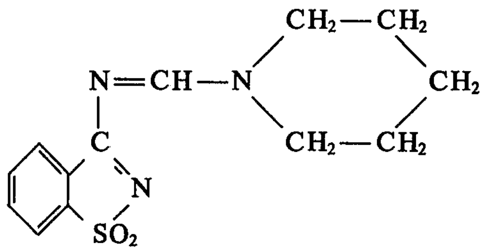

9. Agent selon la revendication 6, qui contient comme composant actif le composé de formule

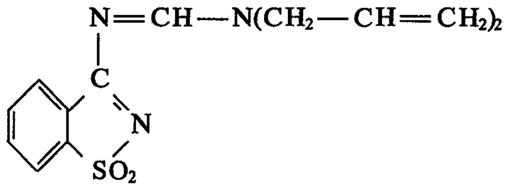

10. Agent selon la revendication 6, qui contient comme composant actif le composé de formule

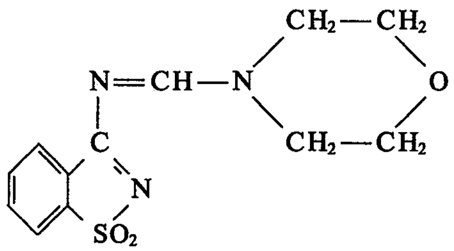

11. Agent selon la revendication 6, qui contient comme composant actif le composé de formule

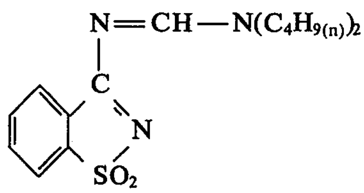

12. Agent selon la revendication 2, qui contient comme composant actif le composé de formule

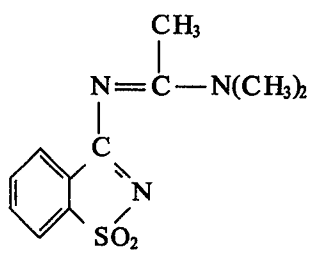

13. Agent selon la revendication 5, qui contient comme composant actif le composé de formule

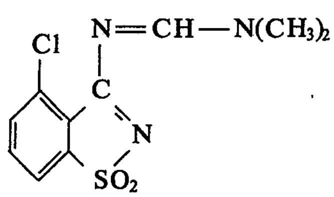

14. Agent selon la revendication 5, qui contient comme composant actif le composé de formule

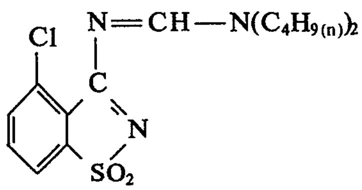

15. Agent selon la revendication 5, qui contient comme composant actif le composé de formule

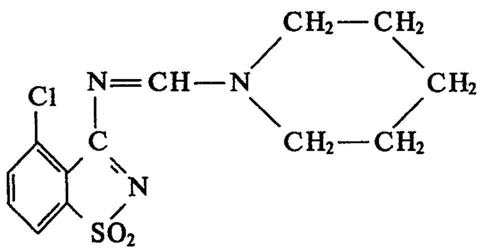

16. Agent selon la revendication 5, qui contient comme composant actif le composé de formule

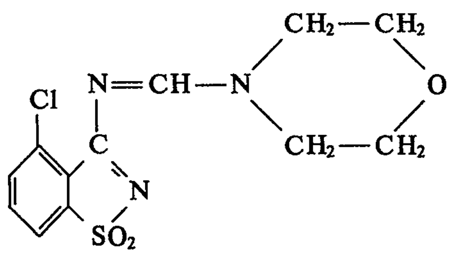

17. Agent selon la revendication 5, qui contient comme composant actif le composé de formule

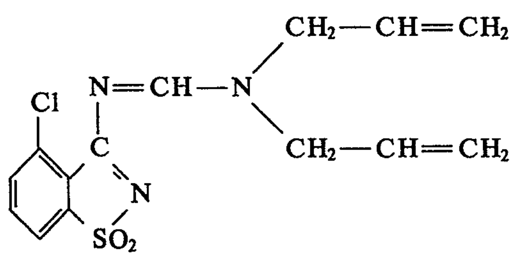

18. Agent selon la revendication 2, qui contient comme composant actif le composé de formule

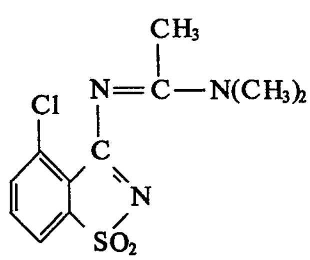

19. Procédé de préparation de 3-amidino-benzisothiazol-1,1-dioxydes de formule

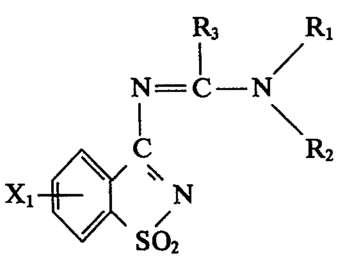

(I)

où

$R_1$ et $R_2$ représentent chacun un alcoyle en $C_1$ à $C_5$ ou un alcényle en $C_2$ à $C_5$,
$R_3$ représente un hydrogène ou un méthyle ou
$R_1$ et $R_2$ représentent ensemble une chaîne alcoylène en $C_3$ à $C_6$ éventuellement interrompue par O, S ou NH ou
$R_1$ et $R_3$ représentent ensemble $-CH_2-CH_2-CH_2-$ et
$X_1$ représente un hydrogène, un halogène, un alcoyle en $C_1$ à $C_5$, un halogène-alcoyle en $C_1$ à $C_5$ ou un alcoxy en $C_1$ à $C_5$,

caractérisé en ce qu'on fait réagir un composé de formule

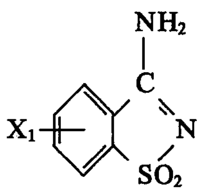

avec un composé de formule

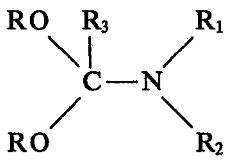

où $R_1$, $R_2$, $R_3$ et $X_1$ ont la signification donnée cidessus et R représente un alcoyle en $C_1$ à $C_5$.

20. Application d'un agent selon la revendication 1, pour combattre les parasites des animaux et des végétaux.

21. Application selon la revendication 21 à la lutte contre les insectes et les représentants de l'ordre des acariens.